# EUROPEAN PATENT SPECIFICATION

(11) **EP 1 370 245 B1**
(45) Date of publication and mention of the grant of the patent: **11.05.2005**
(21) Application number: 02701873.8
(22) Date of filing: 06.03.2002
(51) Int. Cl.: A61K 9/16, A61K 31/138, A61P 9/00

(54) **METHOD TO PREPARE MICROPARTICLES CONTAINING METOPROLOL**
VERFAHREN ZUR HERSTELLUNG VON METOPROLOL ENTHALTENDEN MIKROPARTIKELN
PROCEDE DE PREPARATION DE MICROPARTICULES CONTENANT DU METOPROLOL

(30) Priority: 09.03.2001 SE 0100824
(43) Date of publication of application: 17.12.2003
(73) Proprietor: AstraZeneca AB, 151 85 Södertälje (SE)
(72) Inventor: DJURLE, Alf, S-151 85 Södertälje (SE); HANSSON, Mikael, S-431 83 Mölndal (SE); SÖDERBOM, Malin, S-431 83 Mölndal (SE)
(74) Representative: Bill, Kevin
(86) International application number: PCT/SE2002/000401
(87) International publication number: WO 2002/072074

(56) References cited:
- EP-A1- 0 220 143
- EP-A1- 0 987 020
- WO-A1-99/18935
- DE-A1- 19 733 094
- US-A- 4 946 654
- US-A- 5 628 800
- US-A- 5 883 047

## Description

### Field of invention

The present invention provides microparticles containing metoprolol and a method of obtaining such microparticles using a fluid-bed granulation technique.

### Background of the invention

The strategy for the pharmaceutical formulation work of a given drug depends on different factors. Ultimately, these factors emanate from 1) the therapeutic needs, 2) the physical and chemical properties of the drug, and 3) the influence from the biological environment where the formulation should release its contents. Thus, both technical and biopharmaceutical considerations will contribute to a successful therapy.

Of special importance to the present invention is formulating microparticles containing metoprolol. Such a formulation contains a multitude of discrete delivery units that can be coated with a semipermeable or other polymeric film such as a controlled release coating. Several advantages can be obtained with this type of formulation compared to conventional tablets. The small size of the microparticles ensures a fast and predictable emptying from the stomach and controllable plasma levels of the absorbed drug. From a technological point of view, microparticles are more suitable for coating and handling since a technical fault during the process is fatal for single unit formulations but less so for multiple unit formultions comprising micropellets. Also, microparticle formulations are more versatile for use in different dosage strengths.

An ideal method for the preparation of microparticles containing 80-100% metoprolol should be simple, reproducible and rapid. Several different techniques are available for making microparticles (< 1 mm), e.g., fluidized bed granulation, spray-drying, extrusion-spheronization, spray-chilling, emulsion solvent evaporation/extraction and coating of non-pareil spheres among others. A review by Conti et al. STP Pharma. Sci. 7, 331 (1997) discusses the technical aspects of coacervation, spray-drying, emulsion solvent extraction, and emulsion solvent evaporation.

However, existing techniques suffer from one or more drawbacks: In extrusion spheronization and in coating of non-pareil particles it has been difficult to achieve acceptable microparticles in the range of 50 - 400 µm which contain a high drug content. Pellets made by these methods contain significant amounts of inert excipients.

In emulsifion solvent evaporation, an emulsion has to be made which restricts the use of the drug. Another drawback is the toxicity of the solvent used, usually methylene chloride, which can remain in the microparticles after drying.

Despite many different approaches there is no disclosed technique that produces both (i) small microparticles of less than 250 µm containing metoprolol and (ii) microparticles of more uniform size. Small particles of uniform size improve segregation and dose variation during further processing into capsules or tablets. Further, the existing techniques do not incorporate several desirable aspects such as the possibility to produce spherical microparticles of a small size range that are homogeneous, have a high content of metoprolol and sufficient mechanical strength (to, e.g., withstand coating processes) into one single technique.

There are numerous known processes for preparing granular material using fluidized bed apparatuses. An overview of such processes can be found in, e.g., Aulton (Eds) "Pharmaceutics, The science of dosage form design" Churchill Livingstone, 1988. Basically, fluidization is the operation by which solids are transformed into a fluid like state through the suspension in a gas. Such a system is often referred to as a fluid bed. Fluidized beds are often used for granulation or coating of a product. Granulation is typically performed by spraying droplets of a liquid on particles, which are kept in a fluidized state. The liquid which is sprayed in wets the surface of the solid particles and then solidifies by drying on, or cooling down. In this way, particles grow. Coating is usually performed by spraying a solution of coating agents onto the particles.

A process for preparing granules using a fluidized bed process was presented in US Patent No. 4,946,654. Here, however, there is no teaching regarding how to prepare homogeneous microparticles with at least 80% by weight of metoprolol. WO 99\59544 describes a method of producing granules using a fluidized bed process. The granules were prepared by using a sugar nucleus and then coating the sugar nucleus with the agent of interest and an enteric coating. The average particle diameter of the granules is between 300-400 µm. The application fails to teach to how to prepare homogeneous microparticles which contain at least 80% by weight of metoprolol.

U.S. Patent No. 4,927,640 discloses a controlled release preparation which includes small insoluble particles (cores) which are covered by a pharmaceutically active compound. The cores have the size of 0.1-2 mm and are made from inert insoluble material such as silicon dioxide, glass or plastic.

U.S. Patent No. 4,957,745 discloses a controlled release preparation which includes small compact particles of metoprolol coated with a polymeric membrane comprising derivatives of cellulose without protolysable groups. The small particles containing metoprolol have a size of 0.25 mm-2 mm.

### Object of the invention

An object of the present invention is to provide a method for preparing a homogeneous microparticle of metoprolol, or a salt thereof, or one of its single enantiomers, or a salt thereof, which has a size distribution of less than 250 µm preferably 50 - 200 µm. Another object is to provide a method for preparing a microparticle with high amounts of metoprolol in a high-yield process, e.g., provide homogeneous microparticles with at least 80 weight % of metoprolol, e.g., 85-100 weight %, 90-100 weight %, or 95-100 weight %. Also, the invention provides a method of preparing a homogeneous microparticle with incorporated metoprolol that has low friability and sufficient mechanical strength, such that the microparticle can endure coating and compressing processes.

As used herein, "metoprolol" is intended to mean metoprolol, or a salt thereof, or one of its single enantiomers, or a salt thereof, or a mixture of enantiomers or enantiomer salts.

### Disclosure of the invention

It has been found that spherical, free-flowing, homogeneous microparticles containing at least 80 weight % of metoprolol having low friability can be obtained by spraying a suspension/solution/emulsion containing metoprolol into a fluidized bed thereby forming microparticles having an appropriate size, e.g., particles with a size distribution of less than 250 µm, preferably a size distribution of between 50-200 µm, and selecting out these microparticles from the fluidized bed. The microparticles produced by the method described herein are nearly spherical in shape, have a smooth surface and have a narrow grain spectrum. These characteristics ensure that the microparticles can be coated easily.

More specifically, the method of the present invention includes spraying into droplets a granulation liquid medium containing metoprolol into a fluidized bed. The liquid medium can contain a salt of metoprolol, e.g., metoprolol succinate or metoprolol fumarate, dissolved in a liquid medium, e.g., water. Optionally, a polymer and\or dispersing agent may be added. The solid content of the liquid medium can be in the range between 15 to 60 weight%. The content of metoprolol is at least 80 weight % of the weight of the dried microparticles. Perferably, the weight of the microparticle comprises 80-100% metoprolol. For example, the microparticles can contain 85-100 weight %, 90-100 weight %, or 95-100 weight % metoprolol. If a polymer is added, the polymer can be a water soluble or non-water soluble polymer. Preferably, the polymer is a water soluble polymer. The polymer and\or dispersing agent used in the present invention can act as a binder and plastizer, and can be any polymer or dispersing agent known in the art, e.g., a cellulose derivative, e.g., hydroxypropyl methyl cellulose (HPMC), a polysaccharide, a natural polymer, a synthetic polymer, a surfactant and mixtures thereof. The liquid in which the polymer is soluble can be water, tertiary butyl alcohol, cyclohexane, methylene chloride, methanol, ethanol and mixtures thereof.

It was surprisingly found that microparticles with a very small size distribution of less than 250 µm can be produced and that these microparticles can withstand coating and compressing processes. In one embodiment, the particles selected have a size distribution of between 50 µm to 100 µm, 100 µm to 150 µm, 100 µm to 200, or 150 µm to 240 µm. Particles produced by the method described herein can be coated with one or more polymeric films, e.g., a polymeric layer that allows for controlled release of metoprolol.

Unless otherwise defined, all technical and scientific terms used herein have the same meaning as commonly understood by one of ordinary skill in the art to which this invention belongs. In the case of conflict, the present invention, including definitions will control.

### Metoprolol

Metoprolol is a selective beta-receptor blocking agent (see U.S. Patent No: 3,998,790).

### Metoprolol has the following structure:

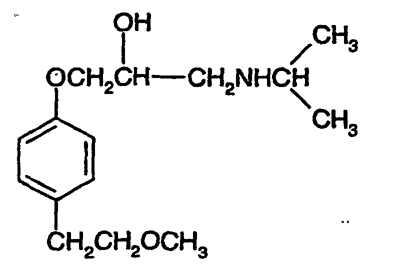

The present invention is related to microparticles of metoprolol or a pharmaceutically acceptable salt thereof, a pharmaceutical composition containing microparticles of metoprolol, methods of making such microparticles and compositions thereof, and methods of using the microparticles for treating cardiovascular disorders. As used herein, "metoprolol" is intended to mean metoprolol, or a salt thereof, or one of its single enantiomers, or a salt thereof, or a mixture of enantiomers or enantiomer salts, e.g., s-isomer can be used. Suitable pharmaceutically acceptable salts of metoprolol include the tartrate, succinate, fumarate or benzoate salts and especially the succinate salt can also be used. The s-enantiomer of metoprolol or a salt thereof, particularly the benzoate salt or the sorbate salt, may also be used.

### Polymers and\or dispersing agents

The polymer and dispersing agent can be, but are not limited to, the excipients listed below:
- *cellulose derivatives,* like ethylcellulose, hydroxypropyl methyl cellulose, hydroxyethyl cellulose, hydroxypropyl cellulose, ethyl hydroxyethyl cellulose, carboxymethyl cellulose, cellulose acetate butyrate, cellulose acetate phtalate, methylcellulose, etc
- *other polysaccharides*, like alginate; xanthan; carrageenan; scleroglucan; pullulan; dextran; hyaluronic acid; chitin; chitosan; starch; etc
- *other natural polymers,* like proteins (e g albumin, gelatin, etc); natural rubber ; *gum arabic; etc*
- *synthetic polymers*, like acrylates (e g polymethacrylate, poly(hydroxy ethyl methacrylate), poly(methyl-methacrylate), poly(hydroxy ethyl methacrylate - co methyl methacrylate), Carbopol® 934, etc); polyamides (e g polyacrylamide, poly(methylen bisacrylamide), etc); polyanhydrides (e g poly(bis carboxyphenoxy)methane, etc); PEO-PPO block-co-polymers (e g poloxamers, etc); polyvinyl chloride; polyvinyl pyrrolidone; polyvinyl acetate; polyvinyl alcohol; polyethylene, polyethylene glycols and co-polymers thereof; polyethylene oxides and co-polymers thereof; polypropylene and co-polymers thereof; polystyrene; polyesters (e g poly(lactid acid), poly(glycolic acid), poly(caprolactone), etc, and co-polymers therof, and poly(ortho esters), and co-polymers thereof); polycarbonate; cellophane; silicones (e g poly (dimethylsiloxane), etc); polyurethanes; synthetic rubbers (e g styren butadiene rubber, isopropene rubber, etc); etc
- *surfactants*, i.e., anionic, like sulphated fatty alcohols (e g sodium dodecyl sulphate), sulphated polyoxyethylated alcohols or sulphated oils, etc; cationic, like one from the group of quaternary ammonium and pyridinium cationic surfactants, etc; non-ionic, like one from the group of polysorbates (e g Tween), sorbitan esters (e g Span), polyoxyethylated linear fatty alcohols (e g Brij), polyoxyethylated castor oil (e g Cremophor), polyoxyethylated stearic acid ( e g Myrj), etc; etc
- *other substances,* like shellacs; waxes (e g carnauba wax, beeswax, glycowax, castor wax, etc); nylon; stearates (e g glycerol palmitostearate, glyceryl monostearate, glyceryl tristearate, stearyl alcohol, etc); lipids (e g glycerides, phospholipids, etc); paraffin; lignosulphonates; mono- or disaccharides (e.g. lactose, etc.); sugar alcohols (e.g. mannitol etc.); etc.

Also, combinations of these excipients are possible.

The excipients mentioned above may be made more ductile by introducing a plasticizer. The plasticizer could be but is not limited to the plasticizers mentioned below:
- glycerol, polyethylene glycol, propylene glycol, triethyl citrate, diethyl phthalate, dibutyl phthalate, dibutyl sebacate, sorbitol, triacetin, etc.

Also, combinations of these plasticizers are possible.

### Low friability microparticles containing metoprolol

Generally the following conditions are used to obtain low friability microparticles according to the method of the invention.

To obtain low friability microparticles the solid content of the suspension/solution/emulsion should be high, preferably in the range 10-60 weight %, more preferably 20 - 60 weight %. Expressed otherwise, low friability microparticles, that can for instance endure coating with a polymeric film, are achieved when the suspension/solution/emulsion has a solid volume content equal to or higher than 15 vol%, preferably up to 60 vol %. A microparticle having a high total content of metoprolol can be obtained, for example, as much as 80 weight %, e.g., 85 weight %, 90 weight %, or 100 weight % (based upon solid content). The median pore size of the obtained microparticles is preferably less than 1.0 µm. Solid content and solid volume content are weight % and volume %, respectively, of dry material in the suspension/solution/emulsion (dry/(dry + liquid)), wherein the dry material is metoprolol (and optionally also a polymer and\or dispersing agent).

The content of the metoprolol calculated on the weight of the dried microparticles ranges from 80 to 100 weight %, e.g., 85-99 weight %, 90-99 weight %, or 95 -99 weight %.

The solid content of the liquid medium is defined as the residue after drying at 110°C for 2 hours, divided by the total amount before drying. The solid content can be expressed either as weight percent or preferably as volume percent.

A microparticle according to the present invention comprises metoprolol, with optionally one (or more) additional active or non-active substances, which are dispersed within the microsphere.

### Methods of making microparticles

The spherical, free-flowing, homogeneous microparticles described herein can be obtained using any known fluidized bed granulation process, e.g., as described in U.S. Patent No. 4,946,654. A preferred method of forming the homogeneous microparticles includes using a continuous fluid-bed granulation process which has an integrated microparticle selecting system that selects microparticles having a desired size distribution, e.g., microparticles having a size distribution of less than 250 µm. In such a continuous fluid-bed granulation process, there is an external equilibrium between the supply of granulation liquid and the discharge of microparticles and the internal equilibrium between the granulation and nucleation processes. Both equilibrium states are directly related to each other. On the side of granulation liquid supply, the optimal spraying of the granulation liquid creates the condition for granulation and nucleation to take place, on the side of the microparticles selection, deliberate continuous selection ensures that only microparticles of the desired grain size are removed from the process.

The following general steps of the procedure are further exemplified in the experimental section.
*a*) Preparation of a granulation liquid medium with a high solid content for atomizing. The medium is a suspension, a solution or an emulsion of metoprolol. Preferably, the medium is a supersaturated solution of metoprolol. Optionally, a polymer and\or dispersing agent can be added. The polymer can act as a binder between the fine active substance particles in the microparticles and can be either a water soluble or a non-water soluble polymer.
*b*) Spraying metoprolol-containing suspension/solution/emulsion into a fluidized bed. The suspension/solution/emulsion is fed by a peristaltic pump through one or more spray nozzles, e.g., a pneumatic nozzle, an ultrasonic nozzle, a rotary atomizer or a pressurized nozzle. If two spray nozzles are used, the liquid medium and the air can be mixed outside the nozzle. The atomization gas used can be any gas which is inert under the operating conditions. Generally, the desired spray droplet diameter is of the order of 50 µm.
   In the fluid-bed granulation process, a bottom-up flow of air or inert gas fluidizes the solid metoprolol particles. In the fluidized state, the solid particles are separated from each other and can be wetted with granulation liquid medium all around. If a spray droplet hits a particle, the granulation liquid medium spreads over the surface of the particle, ideally forming a complete liquid film. The intensive exchange of heat and matter between the solid particles and the gas stream accelerates drying and aids the solidification of the liquid film on the surface all over the particle. The repeated application and solidification of the liquid spray causes the particle to grow by layers and form a microparticle. The microparticle is compact and also nearly spherical.
   The growth of particles starts in the fluidized bed from nuclei. Thus, for the granulation process to start, the fluidized-bed apparatus can already contain starting granulate, e.g., crystalline particles of metoprolol. However, it is possible to start granulation in an empty fluidized-bed apparatus. In this embodiment, a spray droplet can be sprayed into an empty fluidized-bed apparatus. Once dry, the droplet can serve as a nucleus.
   Nuclei can be constantly formed in the fluidized bed. For example, spray droplets containing metoprolol which fail to hit a solid particle or reach a particle whose layer has already solidified (spray drying) so that the droplet does not stick on collision with the particle, can serve as a nucleus. In another embodiment, nuclei can be formed by interparticular collision, abrasion and destruction of particles. For example, dust produced following the collision of two solid particles serves as a nucleus for new particle growth.
*c*) Selecting out a microparticle that has a desired size distribution, e.g., of less than 250 µm, e.g., a microparticle around 50 µm, 100 µm or 200 µm. The microparticles of a desired size are selected from the fluidized bed using any known method of selecting out a microparticle from a fluidized bed. In one example, the microparticle is selected out using a countercurrent gravity classifier. For example, the microparticles can be selected using a zigzag classifier. The classifier allows very precise control of the grain size by means of a classifying air stream. The micropellet entering the classifier, forced by gravity, moves downwards on the bottom wall of the classifying duct. At every bend of the classifying duct, the material must pass through the classifying air flow to reach the opposite wall. On its way, the micropellet moves essentially in vertical direction to the classifying air flow. Consequently, across-flow classification occurs at every bend of the duct. Much of the finer micropellet stream with slow floating speed is forced out of the granular stream and carried upwards. To make separation complete, the selection process occurs at several successive bends of the duct. Particles that are eliminated from the discharged material are carried upwards and depending on their size, enter the bed again at shorter or greater distance from the nozzle. Hence, the smaller and lighter particles enter the bed at greater distance from the nozzle. The larger particles are classified and sprayed more often until their size allows them to pass the classifier on the way down.
   Optional step:
d) The metoprolol microparticles described herein can be coated with a polymeric layer, e.g., a polymeric layer that allows for controlled release of metoprolol. The controlled release polymer can be dissolved in water or in an organic solvent such as ethanol, isopropyl alcohol and/or methylene chloride. The spraying can be carried out in a coating pan, but is preferably carried out in a fluidized bed. Ethyl cellulose or other known controlled release polymers can also be applied from an aqueous dispersion (latex).

### Mechanical strength of the microparticles

The microparticles produced by the present method have good mechanical strength and can endure coating and compressing processes. In one embodiment, the dry microparticles are coated with a controlled release coating and dispensed into capsules, or incorporated into a tablet by methods known to those skilled in the art. In another embodiment, the microparticles are compressed into tablets and the tablets are then coated.

### Formulating and administering the microparticles

The microparticles described herein can be formulated into pharmaceutical compositions by admixing with pharmaceutically acceptable nontoxic excipients and carriers. Such compositions can be prepared for administration by various routes, but preferably the composition should be administered orally. The microparticles can be processed into liquid dosage forms and solid dosage forms.

Examples of liquid dosage forms can include pharmaceutically acceptable emulsions, microemulsions, solutions, suspensions, syrups and elixirs. The liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils) glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Solid dosage forms for oral administration include capsules, tablets, pills, effervescent tablets, powders, and granules. In such solid dosage forms, the microparticles described herein can be mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, 3) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and I) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

In one embodiment, the microparticles described herein are processed into a tablet which has fast dissolving\disintegrating properties in the oral cavity, or which can dissolve rapidly\disintegrate in water before being orally administered.

During treatment with metoprolol, it is advantageous to have a constant concentration of metoprolol in the blood. Thus, controlled release of the drug over a long period of time is desireable. Microparticles are therefore perferably coated with a controlled release polymeric coating. Perferably, the microparticles are coated with a controlled release coating which releases the metoprolol with a rate virtually independent of pH over a 16-24 hour time period.

The particles can be coated with any controlled release polymeric coating known in the art. For example, the microparticles can be coated as described in US. Patent No. 4,957,745, with a polymeric membrane containing derivatives of cellulose without protolysable groups. Examples of suitable polymeric materials are ethyl cellulose or a mixture of ethyl cellulose and hydroxypropylmethyl cellulose, hydroxypropyl cellulose, Eudragit RL or Eudragit RS.

Plasticizers and/or pigments may be added to the polymeric layer in order to improve the technical properties of the layer or change the permeability of the coating. Examples of suitable plasticizers are citrate esters, acetylated monoglycerides and glyceryl triacetate, especially preferred is acetyltributylcitrate.

Each coated microparticle of metoprolol forms an individual controlled release unit, releasing the drug at a predetermined rate. Therefore, the coated microparticles make it possible to formulate and administer the preparation in different dosage forms and strengths. They can be filled into, e.g. hard gelatin capsules or sachets or compressed to tablets and still give the desired plasma concentration profile and duration of the effect.

When the coated microparticles of metoprolol are formulated into tablets they are mixed with additives e.g. microcrystalline cellulose, which improves the tabletting properties and facilitates disintegration of the tablet.

### Uses of the microparticles containing metoprolol

Metoprolol can be used to prevent or treat cardiovascular disorders. For example, metoprolol can be used to prevent or treat acute myocardial infarction, congestive heart failure, cardiac arrhythmias, such as atrial, supraventricular and ventricular ectopy, tachycardia, flutter or fibrillation, including atrial, supraventricular and ventricular arrhythmias resulting from myocardial ischemic injury, hypertension, e.g., in particular moderate hypertension and angina pectoris.

The typical daily dose of the metoprolol microparticle composition varies and will depend on various factors such as the individual requirements of the patients. In general, the daily dose will be in the range of 1-400 mg of metoprolol.

### Working examples

The following examples illustrate different aspects of the invention without limiting the scope.

### Example 1.

### Preparation of particles

Microparticles were prepared in a continuous fluidized bed system (Glatt AGT 150, Weimar, Germany) from a solution of metoprolol succinate. The solution was done by dissolving metoprolol succinate (750 g) into warm water (68°C) (1250 g). Solid content of the suspension was 37.5%w/w. The solution (37.5 w/w% metoprolol succinate) was kept in 68°C before further processing.

The solution was warmed up to 75°C and sprayed into a Glatt AGT 150 fluidized bed with a flow rate of 30 g/min. The nozzle had a opening of 0.5 mm. The inlet air flow was approximately 110 m³/h, inlet air temperature 110°C, atomizing air pressure 4 bar, sifter air pressure 75-76 mbar and sifter air flow 1.42 m³/h. Median size of the uncoated particles was 149 µm, 90% smaller than 182 µm and 10% smaller than 113 µm when determined by laser diffractometry. Estimated from scanning electron micrographs, pores on the surface of the particles were smaller than 1 µm.

### Coating of particles

These microparticles (200 g) were coated in a fluidized bed. The composition of coating solution was:

| | |
|---|---|
| Ethylcellulose | 187 g |
| Hydroxypropyl cellulose | 33 g |
| Ethanol | 2224 g |

The agglomerates (> 355 µm) were removed by sieving before compression.

### Compression of particles

The coated microparticles were mixed with microcrystalline cellulose for 10 min in a Turbula mixer (W.A. Bachofen, Switzerland). Sodium stearyl fumarate was then added through a sieve and the final mixture was blended for 2 min. The composition of the mixture is given below:

| | |
|---|---|
| Coated particles | 50.00% |
| Microcrystalline cellulose | 49.85% |
| Sodium stearyl fumarate | 0.15% |

An amount of 591 mg of the mixture, corresponding to a drug content of 95 mg, was individually weighed for each tablet on an analytical balance and manually filled into the die of a single punch press (Korsch EK 0, Germany). Compaction was then performed in the single punch press equipped with 11.3 mm flat-faced punches at maximum compaction forces of 8 and 16 kN. The hardness obtained was 70 and 140 N (Schleuniger, Switzerland) respectively. Approximately 6% of the drug were released after 20h (phosphate buffer with a pH of 6.8 at 37°C) irrespective of the compaction force used.

### Example 2.

### Preparation of particles

Microparticles were prepared in a continuous fluidized bed system (Glatt AGT 150, Weimar, Germany) from a solution of metoprolol succinate. The solution was done by dissolving metoprolol succinate (938 g) into warm water (68°C) (1562 g). Solid content of the suspension was 37.5%w/w. The solution (37.5 w/w% metoprolol succinate) was kept in 68°C before further processing.

The solution was warmed up to 75°C and sprayed into a Glatt AGT 150 fluidized bed with a flow rate of 30 g/min. The nozzle had a opening of 0.5 mm. The inlet air flow was approximately 100 m³/h, inlet air temperature 100°C, atomizing air pressure 4.8 bar and sifter air flow 1.49 m³/h. Median size of the uncoated particles was 118 µm, 90% smaller than 147 µm and 10% smaller than 88 µm when determined by laser diffractometry. Estimated from scanning electron micrographs, pores on the surface of the particles were smaller than 1 µm.

### Coating of particles

These microparticles (200 g) were coated in a fluidized bed. The composition of coating solution was:

| | |
|---|---|
| Ethylcellulose | 68 g |
| Hydroxypropyl cellulose | 12 g |
| Ethanol | 889 g |

The agglomerates (> 250 µm) were removed by sieving before compression.

### Compression of particles

The coated microparticles were mixed with microcrystalline cellulose for 10 min in a Turbula mixer (W.A. Bachofen, Switzerland). Sodium stearyl fumarate was then added through a sieve and the final mixture was blended for 2 min. The composition of the mixture is given below:

| | |
|---|---|
| Coated particles | 50.00% |
| Microcrystalline cellulose | 49.85% |
| Sodium stearyl fumarate | 0.15% |

An amount of 292 mg of the mixture, corresponding to a drug content of approximately 105 mg, was individually weighed for each tablet on an analytical balance and manually filled into the die of a single punch press (Korsch EK 0, Germany). Compaction was then performed in the single punch press equipped with 10 mm level and convex shaped punches at maximum compaction force of 8 and 16 kN. The hardness obtained was 60 and 150 N (Schleuniger, Switzerland) respectively. Approximately 80% of the drug were released after 1h (phosphate buffer with a pH of 6.8 at 37°C) irrespective of the compaction force used.

## Claims

1. A method of preparing a homogeneous microparticle of metoprolol the method comprising:
providing a granulation liquid medium comprising a solid content of metoprolol;
spraying the granulation liquid medium into a fluidized bed; and
selecting out a microparticle that has a size distribution of less than 250 µm, thereby obtaining a dry, homogeneous microparticle having at least 80% by weight of metoprolol.

2. A method according to claim 1 wherein the granulation liquid medium further comprises a polymer and\or dispersing agent.

3. A method according to claim 1, wherein the granulation liquid medium further comprises:
a polymer selected from the group consisting of a water soluble or water insoluble polymer, wherein the polymer is at least 5% by weight based on the solid content, and
a liquid in which the polymer is soluble.

4. A method according to claim 2 wherein the liquid in which the polymer is soluble is selected from the group consisting of water, tertiary butyl alcohol, cyclohexane, methylene chloride, methanol, ethanol and mixtures thereof.

5. A method according to the preceding claims wherein the percentage weight of metoprolol is in the range from 90-100%.

6. A method according to any of the preceding claims wherein the desired size distribution of the microparticle is between 50µm to 100µm.

7. A method according to any of the preceding claims wherein the desired size distribution of the microparticle is between 100 to 200 µm.

8. A method according to any of the preceding claims wherein the solid content of the granulation liquid medium is from 15 to 60 weight %.

9. A method according to any of the preceding claims wherein the solid content of the granulation liquid medium is from 20 to 50 weight %.

10. A method according to any of the preceding claims wherein the granulation liquid medium is a suspension.

11. A method according to any of the preceding claims wherein the granulation liquid medium is a solution.

12. A method according to any of the preceding claims wherein the granulation liquid medium is an emulsion.

13. A method according to any of the preceding claims wherein metoprolol is in a salt form and is selected from the group consisting of metoprolol succinate and metoprolol fumarate.

14. A method according to any of the preceding claims wherein the method further comprises coating the selected microparticle with a controlled release coating.

15. A microparticle prepared according to the method of any of claims 1-14.

16. A homogeneous microparticle comprising metoprolol, wherein the microparticle comprises at least 80% by weight, based on the dry weight of the microparticle, of metoprolol, a salt thereof, one of its single enantiomers, or a salt thereof, and comprises a size distribution of between 50-200 µm.

17. A homogenous microparticle according to claim 16, wherein the microparticle comprises 85-100 % by weight of metoprolol.

18. A microparticle according to claim 16 comprising a size distribution in the range from 50 to 100 µm.

19. A microparticle according to claim 16 comprising a size distribution in the range from 100 to 200 µm.

20. A microparticle according to claim 16 further comprising a controlled release coating.

21. A microparticle according to claim 16 wherein metoprolol is in a salt form and is selected from the group consisting of metoprolol succinate and metoprolol fumarate.

22. A pharmaceutical composition comprising the microparticle of claim 16.

23. The use of a microparticle according to claim 16 for the preparation of a medicament for the prophylaxis or treatment of a cardiovascular disorder.

## Patentansprüche

1. Verfahren zur Herstellung eines homogenen Metoprolol-Mikroteilchens, bei dem man:
ein Granulierflüssigkeitsmedium, das einen Metoprolol-Feststoffgehalt enthält, bereitstellt;
das Granulierflüssigkeitsmedium in eine Wirbelschicht einsprüht und
ein Mikroteilchen mit einer Größenverteilung von weniger als 250 µm ausselektiert, wodurch man ein trockenes, homogenes Mikroteilchen mit mindestens 80 Gew.-% Metoprolol erhält.

2. Verfahren nach Anspruch 1, bei dem das Granulierflüssigkeitsmedium ferner ein Polymer und/oder Dispergiermittel enthält.

3. Verfahren nach Anspruch 1, bei dem das Granulierflüssigkeitsmedium ferner:
ein Polymer aus der Gruppe bestehend aus einem wasserlöslichen oder wasserunlöslichen Polymer, wobei das Polyer mindestens 5 Gew.-%, bezogen auf den Feststoffgehalt, ausmacht, und
eine Flüssigkeit, in der das Polymer löslich ist,
enthält.

4. Verfahren nach Anspruch 2, bei dem man die Flüssigkeit, in der das Polymer löslich ist, aus der Gruppe bestehend aus Wasser, tert.-Butylalkohol, Cyclohexan, Methylenchlorid, Methanol, Ethanol und Mischungen davon auswählt.

5. Verfahren nach einem der vorhergehenden Ansprüche, bei dem der Gewichtsprozentanteil an Metoprolol im Bereich von 90-100% liegt.

6. Verfahren nach einem der vorhergehenden Ansprüche, bei dem die gewünschte Größenverteilung des Mikroteilchens zwischen 50 µm und 100 µm liegt.

7. Verfahren nach einem der vorhergehenden Ansprüche, bei dem die gewünschte Größenverteilung des Mikroteilchens zwischen 100 µm und 200 µm liegt.

8. Verfahren nach einem der vorhergehenden Ansprüche, bei dem der Feststoffgehalt des Granulierflüssigkeitsmediums 15 bis 60 Gew.-% beträgt.

9. Verfahren nach einem der vorhergehenden Ansprüche, bei dem der Feststoffgehalt des Granulierflüssigkeitsmediums 20 bis 50 Gew.-% beträgt.

10. Verfahren nach einem der vorhergehenden Ansprüche, bei dem es sich bei dem Granulierflüssigkeitsmedium um eine Suspension handelt.

11. Verfahren nach einem der vorhergehenden Ansprüche, bei dem es sich bei dem Granulierflüssigkeitsmedium um eine Lösung handelt.

12. Verfahren nach einem der vorhergehenden Ansprüche, bei dem es sich bei dem Granulierflüssigkeitsmedium um eine Emulsion handelt.

13. Verfahren nach einem der vorhergehenden Ansprüche, bei dem Metoprolol in einer Salzform vorliegt und aus der Gruppe bestehend aus Metoprololsuccinat und Metoprololfumarat ausgewählt wird.

14. Verfahren nach einem der vorhergehenden Ansprüche, bei dem man ferner das selektierte Mikroteilchen mit einem Überzug zur kontrollierten Wirkstofffreigabe versieht.

15. Nach dem Verfahren gemäß einem der Ansprüche 1-14 hergestelltes Mikroteilchen.

16. Homogenes, Metoprolol enthaltendes Mikroteilchen, das mindestens 80 Gew.-%, bezogen auf das Trockengewicht des Mikroteilchens, Metoprolol, eines Salzes davon, eines seiner Einzelenantiomere oder eines Salzes davon enthält und eine Größenverteilung zwischen 50-200 µm aufweist.

17. Homogenes Mikroteilchen nach Anspruch 16, das 85-100 Gew.-% Metoprolol enthält.

18. Mikroteilchen nach Anspruch 16 mit einer Größenverteilung im Bereich von 50 bis 100 µm.

19. Mikroteilchen nach Anspruch 16 mit einer Größenverteilung im Bereich von 100 bis 200 µm.

20. Mikroteilchen nach Anspruch 16, das ferner einen Überzug zur kontrollierten Wirkstofffreigabe aufweist.

21. Mikroteilchen nach Anspruch 16, bei dem Metoprolol in einer Salzform vorliegt und aus der Gruppe bestehend aus Metoprololsuccinat und Metoprololfumarat ausgewählt ist.

22. Pharmazeutische Zusammensetzung, enthaltend das Mikroteilchen gemäß Anspruch 16.

23. Verwendung eines Mikroteilchens gemäß Anspruch 16 zur Herstellung eines Arzneimittels für die Prophylaxe oder Behandlung einer kardiovaskulären Erkrankung.

## Revendications

1. Méthode de préparation d'une microparticule homogène de métoprolol, la méthode comprenant :
la fourniture d'un milieu liquide de granulation comprenant une teneur en matière sèche de métoprolol ;
la pulvérisation du milieu liquide de granulation dans un lit fluidisé ; et
la sélection d'une microparticule ayant une répartition granulométrique inférieure à 250 µm, obtenant ainsi une microparticule homogène et sèche ayant au moins 80% en poids de métoprolol.

2. Méthode selon la revendication 1, dans laquelle le milieu liquide de granulation comprend en outre un polymère et/ou un agent de dispersion.

3. Méthode selon la revendication 1, dans laquelle le milieu liquide de granulation comprend en outre : un polymère choisi parmi le groupe constitué par un polymère hydrosoluble ou insoluble dans l'eau, où le polymère constitue au moins 5% en poids sur la base de la teneur en matière sèche, et un liquide dans lequel le polymère est soluble.

4. Méthode selon la revendication 2, dans laquelle le liquide dans lequel le polymère est soluble est choisi parmi le groupe constitué par l'eau, l'alcool tertio-butylique, le cyclohexane, le chlorure de méthylène, le méthanol, l'éthanol et des mélanges de ceux-ci.

5. Méthode selon les revendications précédentes, dans laquelle le pourcentage pondéral de métoprolol est dans la plage de 90-100%.

6. Méthode selon l'une quelconque des revendications précédentes, dans laquelle la répartition granulométrique désirée de la microparticule va de 50 µm à 100 µm.

7. Méthode selon l'une quelconque des revendications précédentes, dans laquelle la répartition granulométrique désirée de la microparticule va de 100 à 200 µm.

8. Méthode selon l'une quelconque des revendications précédentes, dans laquelle la teneur en matière sèche du milieu liquide de granulation va de 15 à 60% en poids.

9. Méthode selon l'une quelconque des revendications précédentes, dans laquelle la teneur en matière sèche du milieu liquide de granulation va de 20 à 50% en poids.

10. Méthode selon l'une quelconque des revendications précédentes, dans laquelle le milieu liquide de granulation est une suspension.

11. Méthode selon l'une quelconque des revendications précédentes, dans laquelle le milieu liquide de granulation est une solution.

12. Méthode selon l'une quelconque des revendications précédentes, dans laquelle le milieu liquide de granulation est une émulsion.

13. Méthode selon l'une quelconque des revendications précédentes, dans laquelle le métoprolol est sous forme de sel et est choisi parmi le groupe constitué par le succinate de métoprolo1 et le fumarate de métoprolol.

14. Méthode selon l'une quelconque des revendications précédentes, dans laquelle la méthode comprend en outre le revêtement de la microparticule sélectionnée par un revêtement à libération contrôlée.

15. Microparticule préparée selon la méthode selon l'une quelconque des revendications 1-14.

16. Microparticule homogène comprenant du métoprolol, dans laquelle la microparticule comprend au moins 80% en poids, sur la base du poids sec de la microparticule, de métoprolol, d'un sel de celui-ci, de l'un de ses énantiomères uniques, ou d'un sel de celui-ci, et comprend une répartition granulométrique comprise entre 50 et 200 µm.

17. Microparticule homogène selon la revendication 16, dans laquelle la microparticule comprend 85-100% en poids de métoprolol.

18. Microparticule selon la revendication 16, comprenant une répartition granulométrique dans la plage allant de 50 à 100 µm.

19. Microparticule selon la revendication 16, comprenant une répartition granulométrique dans la plage allant de 100 à 200 µm.

20. Microparticule selon la revendication 16, comprenant en outre un revêtement à libération contrôlée.

21. Microparticule selon la revendication 16, dans laquelle le métoprolol est sous forme de sel et est choisi parmi le groupe constitué par le succinate de métoprolol et le fumarate de métoprolol.

22. Composition pharmaceutique comprenant la microparticule selon la revendication 16.

23. Utilisation d'une microparticule selon la revendication 16, pour la préparation d'un médicament pour la prophylaxie ou le traitement d'un trouble cardiovasculaire.
